(19) Europäisches Patentamt
European Patent Office
Office européen des brevets

(11) Veröffentlichungsnummer: **0 069 374**
**A2**

(12) # EUROPÄISCHE PATENTANMELDUNG

(21) Anmeldenummer: 82105985.4

(22) Anmeldetag: 05.07.82

(51) Int. Cl.³: C 07 C 39/367
C 07 C 39/44, C 07 C 43/23
A 61 K 31/055, A 61 K 31/085
//C07C37/62, C07C37/11

(30) Priorität: 08.07.81 DD 231562
17.03.82 DD 238211

(43) Veröffentlichungstag der Anmeldung:
12.01.83 Patentblatt 83/2

(84) Benannte Vertragsstaaten:
AT BE CH DE FR GB IT LI NL

(71) Anmelder: VEB Jenapharm
Otto-Schott-Strasse 13
DDR-6900 Jena(DD)

(72) Erfinder: Teubner, Herbert, Dr.
Kantstrasse 40
DDR-3700 Wernigerode(DD)

(72) Erfinder: Kramer, Axel, Dr.
Lutherstrasse 10
DDR-2200 Greifswald(DD)

(72) Erfinder: Weuffen, Wolfgang, Prof.Dr.Dr.
Kirschenweg 9
DDR-2200 Greifswald(DD)

(72) Erfinder: Schrötter, Eberhard, Dr.

DDR-1634 Rangsdorf AWG 21(DD)

(72) Erfinder: Grübel, Gerhard
Hildebrandstrasse 10
DDR-6900 Jena(DD)

(74) Vertreter: Hansen, Bernd, Dr.rer.nat. et al,
Hoffmann . Eitle & Partner Patentanwälte
Arabellastrasse 4
D-8000 München 81(DE)

(54) **Neue Phenolverbindungen, Verfahren zu deren Herstellung und pharmazeutische Mittel, die diese enthalten.**

(57) Die Erfindung betrifft Derivate des Phenols sowie ein Verfahren zu deren Herstellung gemäss der allgemeinen Formel I, in der $R_1$ Brom oder Chlor, $R_2$ Methyl oder Ethyl, $R_3$ Wasserstoff bzw. Natrium, Kalium, Ammonium bzw. substituiertes Ammonium, Magnesium, Kalzium, Barium, Aluminium, Zinn, Wismut, Kupfer oder Zink, $R_4$ und $R_5$ Wasserstoff, Alkyl oder Alkoxy mit bis zu 5 C-Atomen, Brom oder Chlor bedeuten. Die Zielverbindungen werden durch Benzylierung von 6-Halogen- bzw. 4-Halogen-2-alkylphenolen, Halogenierung von 2-Alkyl-4-benzylphenolen bzw. -6-benzylphenolen oder durch Alkylierung von 6-Halogen-4-benzylphenolen bzw. von 4-Halogen-6-benzylphenolen und gegebenenfalls durch Umsetzung der Reaktionsprodukte mit den Hydroxiden, Alkoholaten, Karbonaten oder Hydrogenkarbonaten der oben genannten Metalle erhalten.

Es können wahlweise wasser- oder lipidlösliche Wirkstoffe erhalten werden, die in pharmazeutischen Zubereitungen ein breites antimikrobielles Wirkungsspektrum aufweisen.

EP 0 069 374 A2

HOFFMANN · EITLE & PARTNER
PATENTANWÄLTE
DR. ING. E. HOFFMANN (1930-1976) · DIPL.-ING. W. EITLE · DR. RER. NAT. K. HOFFMANN · DIPL.-ING. W. LEHN
DIPL.-ING. K. FÜCHSLE · DR. RER. NAT. B. HANSEN
ARABELLASTRASSE 4 · D-8000 MÜNCHEN 81 · TELEFON (089) 911087 · TELEX 05-29619 (PATHE)

0069374

37 144 u/wa



# Neue Phenolverbindungen, Verfahren zu deren Herstellung und pharmazeutische Mittel, die diese enthalten

Die Erfindung betrifft neue Phenolverbindungen, Verfahren zu deren Herstellung und pharmazeutische Mittel, die diese enthalten. Die Erfindung bezieht sich insbesondere auf Verbindungen und ein Verfahren zur Herstellung solcher antimikrobiell wirksamer, epidermal verträglicher Verbindungen der allgemeinen Formel I,

worin $R_1$ Brom oder Chlor
$R_2$ Methyl oder Ethyl
$R_3$ H, Natrium, Kalium, Kalzium, Barium, Ammonium, substituiertes Ammonium, Magnesium, Wismut, Zinn, Aluminium, Zink oder Kupfer
$R_4$ und $R_5$ H, Alkyl oder Alkoxy mit bis zu 5 C-Atomen, Brom oder Chlor

bedeuten.

Phenolderivate, wie Chlorphenol, Pentachlorphenol, Kresol, Chlorkresol, Benzylphenol, Benzylchlorphenol, Thymol sind als Antiseptika und Desinfizientia bekannt. Nachteile dieser Verbindungen sind die teilweise hohe Toxizität oder zumindest schlechte Hautverträglichkeit bzw. hohe Sensibilisierungsrate. Es ist ferner bekannt, aus 2-Chlor-6-methylphenol, das als Abfallprodukt bei der Herbizidsynthese anfällt, 2-Chlor-6-methyl-4-benzylphenol herzustellen.

Die Verbindung 2-Chlor-6-methyl-4-benzylphenol ist zwar gut hautverträglich und besitzt trotz einiger

Wirkungslücken allgemein gute antimikrobielle Wirksamkeit. Außerdem stört beim 2-Chlor-6-methyl-4-benzylphenol der Eigengeruch. Schwierigkeiten bei der galenischen Verarbeitung können bei einigen Einsatzgebieten dadurch auftreten, daß 2-Chlor-6-methyl-4-benzylphenol schwer wasserlöslich ist.

Ziel der Erfindung ist, ausgehend von den bekannten guten Eigenschaften des 2-Chlor-6-methyl-4-benzylphenols, Verfahren zu entwickeln und Verbindungen herzustellen, die nicht von der Anwesenheit des Abfallproduktes der Herbizidsynthese abhängig sind, möglichst wenig Eigengeruch besitzen.

Die Gruppe der Zielverbindungen soll sowohl wasserlösliche als auch lipidlösliche Wirkstoffe umfassen. Sie sollen ein möglichst breites antimikrobielles Wirkungsspektrum bei hoher Wirksamkeit aufweisen.

Das Ziel der Erfindung wird dadurch erreicht, daß ausgehend von 2-Alkylphenolen durch Halogenierung nach an sich bekannten Verfahren 2-Halogen-6-alkylphenole hergestellt, diese durch Friedel-Crafts-Benzylierung zu Verbindungen der allgemeinen Formel II mit $R_3$ = H umgesetzt und erforderlichenfalls in Salze der allgemeinen Formel II, in der dann $R_3$ Natrium, Kalium, Kalzium, Barium, Ammonium bzw. substituiertes Ammonium, Magnesium, Wismut, Zinn, Zink, Aluminium oder Kupfer bedeutet, überführt werden.

Zur Herstellung der 6-Halogen-2-alkylphenole wird zweckmäßigerweise das 2-Alkylphenol in 4-Stellung sulfoniert, wodurch selektiv in o-Stellung halogeniert werden kann. Anschließend wird der Sulfonsäurerest hydrolytisch entfernt.

Zur Friedel-Crafts-Benzylierung werden die 6-Halogen-2-alkylphenole in Gegenwart von Friedel-Crafts-Katalysatoren mit Benzylchloriden oder -alkoholen, gegebenenfalls unter Verwendung organischer Lösungsmittel erhitzt.

Als Benzylchloride oder -alkohole kommen neben den unsubstituierten auch durch Alkylreste mit bis zu 5 C-Atomen oder durch Halogene substituierte in Frage.

Das Ziel der Erfindung wird weiterhin dadurch erreicht, daß man ebenfalls von 2-Alkylphenolen ausgeht, die in an sich bekannter Weise in 4-Stellung halogeniert werden und daß die 4-Halogen-2-alkylphenole in ebenfalls an sich bekannter Weise durch Friedel-Crafts-Benzylierung zu Verbindungen der allgemeinen Formel III mit $R_3$ = H umgesetzt und erforderlichenfalls in Salze der allgemeinen Formel III, in der dann $R_3$ Natrium, Kalium, Kalzium, Barium, Ammonium bzw. substituiertes Ammonium, Magnesium, Wismut, Zinn, Zink, Aluminium oder Kupfer bedeutet, überführt werden.

Die Friedel-Crafts-Benzylierung läßt sich mit unsubstituierten Benzylhalogeniden oder -alkoholen oder mit durch vorzugsweise in p-Stellung durch Alkylreste mit bis zu 5 C-Atomen oder durch Halogene substituierten Benzylhalogeniden oder -alkoholen durchführen.

Die Herstellung der genannten Metallsalze erfolgt durch Umsetzung von Verbindungen der allgemeinen Formel I, insbesondere Verbindungen der allgemeinen Formel II und III, in denen $R_3$ = H bedeutet, mit Alkali- oder Erdalkalihydroxiden, mit den entsprechenden Metallalkoholaten, -carbonaten, -hydrogencarbonaten in wäßrigem Medium oder unter Verwendung organischer Lösungsmittel. Bei der Herstellung der Salze erweist sich das Arbeiten unter Inertgas zum Sauerstoffausschluß als zweckmäßig, um stabile farblose Produkte zu erhalten.

Weitere Möglichkeiten zur Herstellung der 6-Halogen-2-alkyl-4-benzylphenole gemäß allgemeiner Formel II ergeben sich durch Halogenierung von 2-Alkyl-4-benzylphenolen sowie durch Alkylierung von 6-Halogen-4-benzylphenolen.

Die Herstellung der 2-Alkyl-4-halogen-6-benzylphenole gemäß allgemeiner Formel III kann ebenfalls durch Halogenierung von 2-Alkyl-6-benzylphenolen in 4-Stellung sowie durch Alkylierung von 4-Halogen-6-benzylphenolen in 2-Stellung erfolgen.

Die erfindungsgemäßen Produkte zeichnen sich durch hohe antimikrobielle Wirksamkeit verbunden mit geringer Toxizität und guter Hautverträglichkeit aus. Dabei weisen die Bromverbindungen einen wesentlich geringeren Eigengeruch auf als die entsprechenden Chlorverbindungen. Es hat sich außerdem gezeigt, daß die antimikrobielle Wirksamkeit der Bromverbindungen die der Chlorverbindungen übertrifft, und daß die Salze im Vergleich mit den freien Phenolen fast geruchlos sind. Beispielsweise ist das Kalziumsalz des 2-Chlor-6-methyl-4-benzylphenols besonders gegen grampositive Bakterien wirksam, während gegen gramnegative Bakterien eine mikrobiostatische Grenzkonzentration von 1 :250 ermittelt wurde. Für praktische Belange ist auf Grund der guten Verträglichkeit eine Anwendungskonzentration bis 1 % ausreichend.

Je nach dem vorgesehenen Verwendungszweck in der Technik oder für pharmazeutische Zwecke können die Verbindungen gemäß allgemeiner Formel I als in Wasser schwerlösliche ölige Flüssigkeiten oder in Form ihrer hydrophilen Salze zur Anwendung gelangen.

Die Erfindung soll anhand nachstehender Ausführungsbeispiele näher erläutert werden.

Beispiel 1

6-Brom-2-methyl-4-benzylphenol

In einem 250 ml Dreihalskolben, ausgestattet mit Rührer, Innenthermometer, Claisen-Aufsatz, Kühler und Trockenröhrchen, werden bei Raumtemperatur 54 g (0,5 mol) 2-Methylphenol (o-Kresol) vorgelegt. Unter Rühren werden 61,0 g konz. $H_2SO_4$ mit 10 % freiem $SO_3$ innerhalb von 35 min zugetropft. Bei der Bildung der 4-Sulfonsäure des Phenols macht sich eine starke Exothermie bemerkbar. Zur Vervollständigung der Reaktion wird anschließend noch 5 Stunden bei 80 °C nachgerührt. Nach dem Abkühlen wird das Reaktionsgemisch auf 900 ml Eiswasser gegossen.

Nach dem Auftauen gibt man alles in einem entsprechend großen Kolben und hierzu werden innerhalb von 1 1/2 Stunden bei 15 bis 27 °C 80 g Brom (0,5 mol) zugetropft. Gegen Ende der Bromzugabe macht sich eine schwache HBr-Entwicklung bemerkbar. Anschließend wird noch 45 min nachgerührt. Das gesamte Reaktionsgemisch wird einer Wasserdampfdestillation mit überhitzten Wasserdampf von 180 bis 200 °C unterzogen, um gleichzeitig den Sulfonsäurerest in 4-Stellung abzuspalten. Somit ist es recht leicht möglich, gezielt aus dem 2-Methylphenol das 6-Brom-2-methylphenol zu synthetisieren, das eine ölige, wasserklare Flüssigkeit darstellt.

Von dem so erhaltenen 6-Brom-2-methylphenol werden 500 g zusammen mit 10 g Zinkspänen auf etwa 100 °C erhitzt. Innerhalb von 20 min werden 453 g Benzylchlorid unter Rühren und unter Feuchtigkeitsausschluß zugetropft. Während weiterer 12 min wird auf 150 °C erhitzt. Das Reaktionsgemisch wird dann insgesamt 5 Stunden bei der Temperatur von 150 °C gehalten. Nach den Erkalten wird vom Zink abgetrennt.

Das Reaktionsprodukt wird in Vakuum bei 0,03 bis 0,04 Torr destilliert. Es werden 305 g 6-Brom-2-methyl-4-benzylphenol als eine schwach gelbliche, ölige Flüssigkeit erhalten, die nahezu geruchlos ist.

Beispiel 2

6-Chlor-2-methyl-4-benzylphenol

Analog Beispiel 1 kann das 6-Chlor-2-methyl-4-benzylphenol hergestellt werden, wenn an Stelle der Bromierung eine Chlorierung vorgenommen wird.

Beispiel 3

6-Brom-2-ethyl-4-benzylphenol

Hierbei gelangen zum Einsatz 61 g (0,5 mol) 2-Ethylphenol, leicht darstellbar aus 2-Ethylanilin, und 61 g konz. $H_2SO_4$ mit 10 % freiem $SO_3$. Die Synthese der 4-Sulfonsäure aus dem 2-Ethylphenol erfolgt analog Beispiel 1; sie verläuft ebenfalls als exotherme Reaktion.

Nach vollständiger Umsetzung wird das Reaktionsgemisch auf 900 ml Eiswasser gegossen und nach dem Auftauen des Eises in einem entsprechend dimensionierten Kolben die Bromierung durchgeführt und gleichzeitig darin die Wasserdampfdestillation mit dem überhitzten Wasserdampf von 180 bis 200 °C zwecks Abspaltung der $SO_3H$-Gruppe vorgenommen. In der Vorlage fällt das 6-Brom-2-ethylphenol als wasserklare, ölige Flüssigkeit an: Kp 96 °C/10 Torr mit einem Brechungsindex: $n_D^{20}$ 1,5631.

Das so gewonnene 6-Brom-2-ethylphenol wird nach an sich bekannten Bedingungen der Friedel-Crafts-Alkylierung mit Benzylchlorid gemäß Beispiel 1 zum 6-Brom-2-ethyl-4-benzylphenol umgesetzt.

Beispiel 4

Darstellung des Natriumsalzes des 6-Chlor-2-methyl-4-benzylphenols

23,2 g (0,1 mol) des 6-Chlor-2-methyl-4-benzylphenols werden in 250 ml Xylol gelöst und unter Rühren wird 5 min ein Stickstoffstrom durch die Apparatur geleitet, um den Luftsauerstoff zu verdrängen. Anschließend wird eine Lösung, bestehend aus 4 g (0,1 mol) NaOH gelöst in einem Zug zugesetzt. Das Reaktionsgemisch wird anschließend ca. 30 min nachgerührt, unter Rühren und weiteren Stickstoffdurchleiten im Vakuum das Lösungsmittel abdestilliert. Als Rückstand fällt das entsprechende Natriumsalz des 6-Chlor-2-methyl-4-benzylphenols an.

Beispiel 5

Natriumsalz des 6-Chlor-2-methyl-4-benzylphenols

23,2 g (0,1 mol) des 6-Chlor-2-methyl-4-benzylphenols werden in 230 ml Tetrachlorkohlenstoff gelöst. Zwecks Ausschluß von Luftsauerstoff erfolgt 5 min ein Durchleiten von Stickstoff durch die Apparatur unter Rühren. Zu dieser Lösung wird eine wäßrige Lösung, bestehend aus 4 g (0,1 mol) NaOH und 5 ml $H_2O$, zügig zugesetzt. Bei einer Temperatur zwischen 20 bis 30 °C wird das Gemisch 1,5 Stunden unter weiterem Stickstoffdurchleiten stark gerührt zwecks Vervollständigung der Reaktion. Nach dieser Zeit werden in Vakuum das Lösungsmittel und das eingesetzte Wasser destillativ abgetrennt. Zur Vervollständigung des Wasserentzuges aus dem Reaktionsgemisch erfolgt wiederholtes Suspendieren des Alkalisalzes mit Tetrachlorkohlenstoff und anschließendes Austreiben des Lösungsmittels im Vakuum. Nach 2 - 3 solchen Arbeitsgängen kann das Alkalisalz als entwässert bezeichnet werden. Der kristallin anfallende Rückstand des Alkalisalzes des 6-Chlor-2-methyl-4-benzylphenols wird zwecks guten Absaugens mit wenig Tetrachlorkohlenstoff angeteigt. Ein Nachwaschen mit dem Lösungsmittel Tetrachlorkohlenstoff schließt sich an, da die Löslichkeit hierin sehr gering ist.

Nach dieser Arbeitsweise läßt sich nahezu quantitativ aus dem 6-Chlor-2-methyl-4-benzylphenol das entsprechende Natriumsalz gewinnen. Ein Waschen mit Diethylether erweist sich zur Verbesserung der farblichen Qualität als zweckmäßig. Die Löslichkeit in Wasser kann als gut bezeichnet werden. Das Natriumsalz des 6-Chlor-2-methyl-4-benzylphenols ist gut wasserlöslich.

Beispiel 6

Darstellung des Kaliumsalzes von 6-Chlor-2-methyl-4-benzylphenols

23,2 g (0,1 mol) des 6-Chlor-2-methyl-4-benzylphenols werden in 250 ml Xylol gelöst. Durch diese Lösung wird unter Rühren 5min Stickstoff zwecks Verjagen des in der Apparatur befindlichen Luftsauerstoffes geleitet. Unter weiterem Rühren und kontinuierlichen Stickstoffdurchleiten wird eine Lösung, bestehend aus 5,6 g (0,1 mol) KOH gelöst in 5 ml $H_2O$, zügig zugefügt. Zwecks Vervollständigung der Reaktion wird 30 bis 60 min nachgerührt und anschließend im Vakuum das Lösungsmittel Xylol abdestilliert, wobei ebenfalls eine Entwässerung einhergeht, da Xylol ein recht gutes Schleppmittel ist. Nach vollständigem Verjagen des Xylol wird das Kaliumsalz in wenig Tetrachlorkohlenstoff suspendiert und abgesaugt. Auch hierbei kann Tetrachlorkohlenstoff bzw. Diethylether aufgrund der geringen Löslichkeit nachgewaschen werden. Die Löslichkeit des Kaliumsalzes in Wasser ist gut.

Analog der Herstellung des Kaliumsalzes in dem Lösungsmittel Xylol kann als Lösungsmittel, worin die Umsetzung durchgeführt werden kann, Aceton oder Tetrachlorkohlenstoff zum Einsatz gelangen.

Beispiel 7

Darstellung des Kalziumsalzes des 6-Chlor-2-methyl-4-benzylphenols

23,2 g (0,1 mol) des 2-Chlor-6-methyl-4-benzylphenols werden in 250 ml Aceton gelöst. Durch diese Lösung wird ein Stickstoffstrom zwecks Verjagen des Luftsauerstoffes unter Rühren geleitet. Zu dieser Lösung wird eine Suspension, bestehend aus 7,4 g (0,1 mol) $Ca(OH)_2$ und 20 ml $H_2O$, zugesetzt. Aufgrund der geringen Löslichkeit von $Ca(OH)_2$ in Wasser erfolgt der Einsatz des doppelten Äquivalents. Das Gemisch wird ca. 60 min bei 20 - 30 °C unter kontinuierlichem Stickstoffdurchleiten durch die Apparatur intensiv gerührt. Die acetonische Lösung enthält das gewünschte Kalziumsalz des 2-Chlor-6-methyl-4-benzylphenols und wird vom überschüssigen Kalziumhydroxid durch Filtration abgetrennt. Das Lösungsmittel Aceton wird anschließend unter Vakuum entfernt. Zwecks weiterer Entwässerung des anfallenden kristallinen Kalziumsalzes wird dieses einige Mal mit Tetrachlorkohlenstoff suspendiert und das Lösungsmittel jeweils im Vakuum mit Anteilen von Wasser abgezogen. Das anfallende Kalziumsalz stellt eine nahezu weiße, sehr stabile Verbindung dar, die jedoch nur gering in Wasser löslich ist.

Analog Beispiel 4 bis 7 können die Alkali- und Erdalkalisalze sowie die Salze des Ammoniums, des Wismuts, Zinns, Zinks, Aluminium und Kupfer von 6-Chlor-2-ethyl-4-benzylphenol hergestellt werden.

Beispiel 8

2-Chlor-6-methyl-4-(4'-tert. butylbenzyl)-phenol

4-tert. Butylbenzylchlorid läßt sich zum Beispiel durch Chlormethylierung von tert. Butylbenzol gewinnen. Dieses substituierte Benzol ist durch Friedel-Crafts-Reaktion aus Benzol und dem tert. Butylhalogenid darstellbar.

106,5 g (0,75 mol) 2-Chlor-6-methylphenol und 45,67 g (0,25 mol) 4-tert. Butylbenzylchlorid werden in 200 ml Chloroform gelöst, 22,5 g wasserfreies Zinkchlorid zum Einleiten der Friedel-Crafts-Reaktion zugesetzt und unter Rühren das Gemisch 5 bis 6 h bei 60 °C gehalten. Nach Abkühlung erfolgt 3maliges Waschen mit je ca. 200 ml Wasser zum Herauslösen des Zinkchlorids aus dem Reaktionsgemisch. Die organische Phase wird über Magnesiumsulfat getrocknet, das Lösungsmittel Chloroform ausgetrieben und anschließend im Vakuum das 2-Chlor-6-methyl-4-(4'-tert. butylbenzyl)-phenol destilliert. Nachdem das überschüssige 2-Chlor-6-methylphenol als Vorlauf übergegangen ist, folgt das substituierte, benzylierte Phenol als nahezu wasserklare dickflüssige, geruchlose Flüssigkeit,

Kp 160 - 165 °C/0,2 Torr

$n_D^{20}$ 1,5635

Analyse

| | | |
|---|---|---|
| Cl | ber.: | 12,3 % |
| | gef.: | 12,11 % |

Ausbeute: 64 %

Beispiel 9

2-Chlor-6-methyl-4-(4'-isopropylbenzyl)-phenol

85,8 g (0,6 mol) 2-Chlor-6-methylphenol und 33,8 g (0,2 mol) 4-Isopropylbenzylchlorid, gelöst in 160 ml Chloroform, werden mit 18 g wasserfreiem Zinkchlorid versetzt. Bei einer Badtemperatur von 60 °C wird das Reaktionsgemisch 5 bis 6 h gerührt, anschließend zwecks Entfernung von überschüssigem Zinkchlorid 3mal mit je 150 ml Wasser gewaschen, getrocknet über Natriumsulfat und das Lösungsmittel Chloroform im Vakuum ausgetrieben.

Die Reinigung des so dargestellten substituierten Benzylphenols erfolgt durch Destillation unter Vakuum.

Als erste Fraktionen gehen die überschüssigen Anteile an 2-Chlor-6-methylphenol über. Bei 146 °C bis 150 °C/ 0,2 Torr destilliert das gewünschte 2-Chlor-6-methyl-4-(4'-isopropylbenzyl)-phenol als nahezu farblose und Substanz. $n_D^{20}$ 1,5719. Bei längerem Stehen beginnt die Substanz zu kristallisieren.

Analyse

| | | |
|---|---|---|
| Cl | ber. | 12,9 % |
| | gef. | 13,14 % |

Ausbeute: 82,5 % d. Th.

Beispiel 10

2-Chlor-6-methyl-4-(3'.4'-dichlorbenzyl)-phenol

90,4 g (0,63 mol) 2-Chlor-6-methylphenol werden mit 149,4 g (0,765 mol) 3.4-Dichlorbenzylchlorid und 2,4 g wasserfreiem, feingemörderten $ZnCl_2$ ohne Lösungsmittel zur Reaktion gebracht. Es wird 5 bis 6 h bei 60 °C gerührt und anschließend aufgearbeitet, indem das Reaktionsgemisch dreimal mit je 100 ml Wasser gewaschen wird.

Vakuumdestillation zur Reinigung des 2-Chlor-6-methyl-4-(3'.4'-dichlorbenzyl)-phenols schließt sich an.
Kp 168 - 169,5 °C/0,16 Torr

Dieses Phenolderivat kristallisiert nach der Destillation aus F 106 - 107 °C

Cl ber. 35,27 %
gef. 35,20 %
35,08 %

Ausbeute: 24,9 % d. Th.

Beispiel 11

2-Chlor-6-methyl-4-(4'-chlorbenzyl)-phenol

178,1 g (1,25 mol) 2-Chlor-6-methylphenol und 80,5 g (0,5 mol) 4-Chlorbenzylchlorid werden in 300 ml getrocknetem Chloroform gelöst. Anschließend erfolgt die Zugabe von 30 g wasserfreiem, feingemörsertem $ZnCl_2$.
Die Friedel-Crafts-Alkylierung setzt sofort ein (HCl-Entwicklung sichtbar).

Das Reaktionsgemisch wird 5 bis 6 h bei 60 °C gerührt, anschließend dreimal mit je 250 ml Wasser gewaschen, das Lösungsmittel Chloroform unter Normaldruck abdestilliert.

Die Reinigung des 2-Chlor-6-methyl-4-(4'-chlorbenzyl)-phenols erfolgt durch Vakuumdestillation; es geht bei Kp 135 - 138 °C/0,02 Torr als etwas gelb-grün gefärbte ölige, geruchlose Flüssigkeit über.

$n_D^{20}$ 1,5990

Cl ber. 26,54 %
gef. 25,53 %
25,48 %

Ausbeute: 47,3 % d. Th.

Beispiel 12

4-Chlor-2-methyl-6-(4'-tert. butylbenzyl)-phenol

106,5 g (0,75 mol) 4-Chlor-2-methylphenol und 45,67 g (0,25 mol) 4-tert. Butylbenzylchlorid werden in 200 ml Chloroform gelöst, 22,5 g wasserfreies Zinkchlorid als Friedel-Crafts-Katalysator werden zugesetzt. Danach wird das Reaktionsgemisch gerührt, wobei schon bei Zimmertemperatur zu Beginn der Reaktion heftige Gasentwicklung, d. h. heftige Reaktion, zu verzeichnen ist. Anschließend wird das Reaktionsgemisch bei 60 °C 5 bis 6 h gerührt und zwecks Entfernen des Zinkchlorids 3mal mit je 200 ml Wasser gewaschen. Die organische Phase wird über Magnesiumsulfat getrocknet und das Chloroform im Vakuum ausgetrieben. Bei 182 - 185 °C/0,3 Torr geht das 4-Chlor-2-methyl-6-(4'-tert. butylbenzyl)-phenol als wasserklare, zähflüssige, nahezu geruchlose Flüssigkeit über.

$n_D^{20}$ 1,5672

Analyse

| | | |
|---|---|---|
| Cl | ber.: | 12,3 % |
| | gef.: | 12,34 % |

Ausbeute: 64 %

Beispiel 13

4-Chlor-2-methyl-6-(4'-isopropylbenzyl)-phenol

4-Chlor-2-methylphenol ist beispielsweise aus 2-Methylphenol (o-Kresol) leicht darstellbar. Ausgehend von diesem substituierten Phenol ist die Alkylierung mittels des substituierten Benzylchlorids, 4-Isopropylbenzylchlorid, durchzuführen. 4-Isopropylbenzylchlorid ist aus Cumol durch Chlormethylierung gut darstellbar.

106,5 g (0,75 mol) 4-Chlor-2-methylphenol und 42,25 g (0,25 mol) 4-Isopropylbenzylchlorid werden in 200 ml Chloroform gelöst, zur Alkylierungsreaktion anschließend 22,5 g wasserfreies Zinkchlorid zugefügt. Das Reaktionsgemisch wird 5 bis 6 h bei 60 °C gerührt und nach Abkühlung wie folgt aufgearbeitet:

Zur Entfernung des Zinkchlorids wird 3mal mit je 200 ml Wasser ausgewaschen und anschließend über Magnesiumsulfat getrocknet. Das Lösungsmittel Chloroform wird im Vakuum entfernt, eine Vakuumdestillation zwecks Reinigung des substituierten Benzylphenols schließt sich an. Das 4-Chlor-2-methyl-6-(4'-isopropylbenzyl)-phenol, eine ölige, nahezu farb- und geruchlose Flüssigkeit, destilliert bei 162 - 165 °C/0,4 Torr über.

$n_D^{20}$ 1,5752

Analyse

| | | |
|---|---|---|
| Cl | ber.: | 12,9 % |
| | gef.: | 12,99 % |

Ausbeute: 50,3 %

Beispiel 14

4-Chlor-2-ethyl-6-(4'-isopropylbenzyl)-phenol

Ausgehend von 2-Ethylphenol, das sich beispielsweise aus dem Nebenprodukt der Chloramphenicolsynthese, dem 2-Nitroethylbenzol, über Reduktion zum 2-Ethylanilin, Diazotierung und Verkochung, leicht darstellen läßt, ist durch Chlorierung das 4-Chlor-2-ethylphenol zugänglich.

117,5 g (0,75 mol) 4-Chlor-2-ethylphenol werden mit 42,25 g (0,25 mol) 4-Isopropylbenzylchlorid in 200 ml Chloroform gelöst. Zur Alkylierungsreaktion werden 22,5 g wasserfreies Zinkchlorid hinzugefügt. Bereits bei 30 °C tritt kräftige Reaktion ein. Das gesamte Gemisch wird 5 bis 6 h bei 60 °C zur Vervollständigung der Alkylierung nachgerührt. Es wird abkühlen gelassen, anschließend mit 3mal je 200 ml Wasser gewaschen, über Natriumsulfat getrocknet und das Lösungsmittel Chloroform ausgetrieben.

Eine Vakuumdestillation zwecks Reinigung des dargestellten substituierten Benzylphenols schließt sich an. Das 4-Chlor-2-ethyl-6-(4'-isopropylbenzyl)-phenol stellt eine farblose, nahezu geruchlose ölige Flüssigkeit dar, Kp 172 - 176 °C/0,6 Torr, $n_D^{20}$ 1,5700

Analyse:

| | |
|---|---|
| Cl | ber.: 12,3 % |
| | gef.: 12,75 % |
| Ausbeute: | 73,6 % d. Th. |

Absolute Hemmung des Wachstums grampositiver Bakterien durch ausgewählte Verbindungen der allgemeinen Formel II

| Verbindung | Toxizität +) | wirksame Verdünnung |
|---|---|---|
| 2-Brom-6-methyl-4-benzylphenol | 1 500 | 1 : 15 000 |
| 2-Brom-6-ethyl-4-benzylphenol | 2 800 | 1 : 60 000 |
| 2-Chlor-6-methyl-4(4'-isopropylbenzyl)-phenol | nicht ermittelt | 1 : 60 000 |
| 2-Chlor-6-methyl-4(4'-tert.Butyl-benzyl)-phenol | nicht bestimmt | 1 : 60 000 |

Vergleiche :

| | | |
|---|---|---|
| 2-Chlor-6-methyl-4-benzylphenol | 1 200 | 1 : 10 000 |

+) Die Toxizität in mg/kg Körpergewicht ist zuverstehen als akute Toxizität an der weißen Maus bei subkutaner Applikation

Absolute Hemmung des Wachstums gram-positiver Bakterien durch ausgewählte Verbindungen der allgemeinen Formel III

| Verbindung | wirksame Verdünnung |
|---|---|
| 4-Chlor-2-methyl-6-(4'-isopropylbenzyl)-phenol | 1 : 500 000 |
| 4-Chlor-2-methyl-6-(4'-tert. Butylbenzyl)-phenol | 1 : 500 000 |

Vergleich:

| | |
|---|---|
| 2-Chlor-6-methyl-4-benzylphenol | 1 : 10 000 |

# Patentansprüche

1. Verbindungen der allgemeinen Formel I,

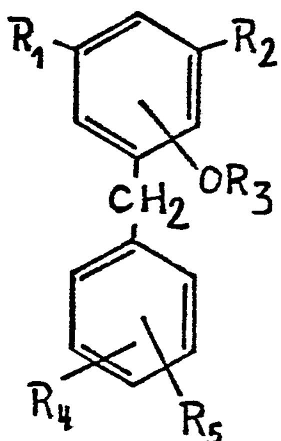

in der $R_1$ Brom oder Chlor
$R_2$ Methyl oder Ethyl
$R_3$ Wasserstoff, Natrium, Kalium, Kalzium, Barium, Ammonium, substituiertes Ammonium, Magnesium, Wismut, Zinn, Zink, Aluminium oder Kupfer
$R_4$ und $R_5$ Wasserstoff, Alkyl oder Alkoxy mit bis zu 5 C-Atomen, Brom oder Chlor
bedeuten

ausgenommen die Verbindung der allgemeinen Formel I,
in der $R_1$ Chlor
$R_2$ Methyl
$R_3$, $R_4$ und $R_5$ Wasserstoff bedeuten.

2. Verbindungen der allgemeinen Formel II

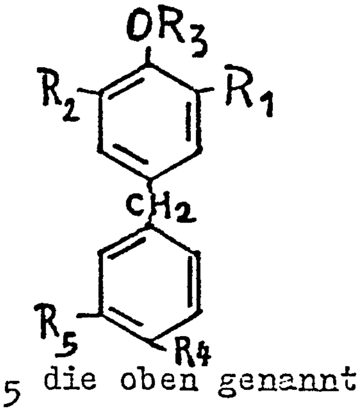

in der $R_1$ bis $R_5$ die oben genannte Bedeutung haben, ausgenommen die Verbindung der allgemeinen Formel II, in der $R_1$ Chlor

$R_2$ Methyl

$R_3$, $R_4$, $R_5$ Wasserstoff

bedeuten.

3. Verbindungen der allgemeinen Formel III

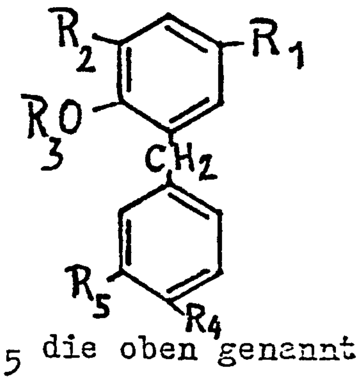

in der $R_1$ bis $R_5$ die oben genannte Bedeutung haben.

4. Verfahren zur Herstellung antimikrobiell wirksamer Derivate des Phenols gemäß Anspruch 1 bis 3, dadurch gekennzeichnet

a) Verbindungen der allgemeinen Formel IV

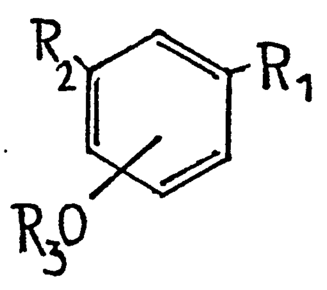

in der $R_1$ und $R_2$ die oben genannte Bedeutung haben und $R_3$ H bedeuten in an sich bekannter Weise benzyliert,

b) Verbindungen der allgemeinen Formel V

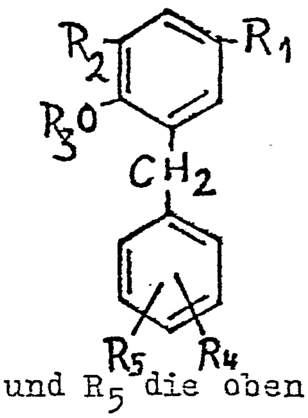

in der $R_2$, $R_4$ und $R_5$ die oben genannte Bedeutung haben und $R_3$ H bedeutet in an sich bekannter Weise halogeniert oder

c) Verbindungen der allgemeinen Formel VI

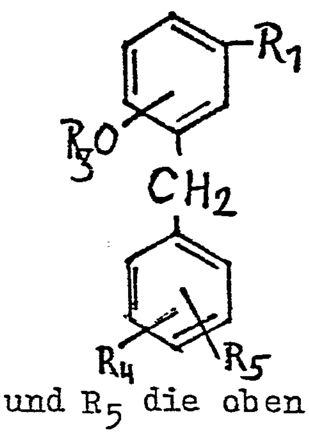

in der $R_1$, $R_4$ und $R_5$ die oben genannte Bedeutung haben und $R_3$ H bedeutet, in an sich bekannter Weise alkyliert werden,

und die so erhaltenen Verbindungen der allgemeinen Formel I in der $R_1$, $R_2$, $R_4$ und $R_5$ die oben genannte Bedeutung haben und $R_3$ H bedeutet gegebenenfalls mit Hydroxiden, Alkoholaten, Karbonaten oder Hydrogenkarbonaten der Metalle Natrium, Kalium, Ammonium bzw. substituiertes Ammonium, Magnesium, Kalzium, Barium, Aluminium, Zinn, Wismut, Kupfer oder Zink umgesetzt werden.

5. Pharmazeutische Mittel, g e k e n n z e i c h - n e t durch einen Gehalt mindestens einer Verbindung nach den Ansprüchen 1 bis 3 nebst üblichen pharmazeutisch annehmbaren Hilfs- und Trägerstoffen.

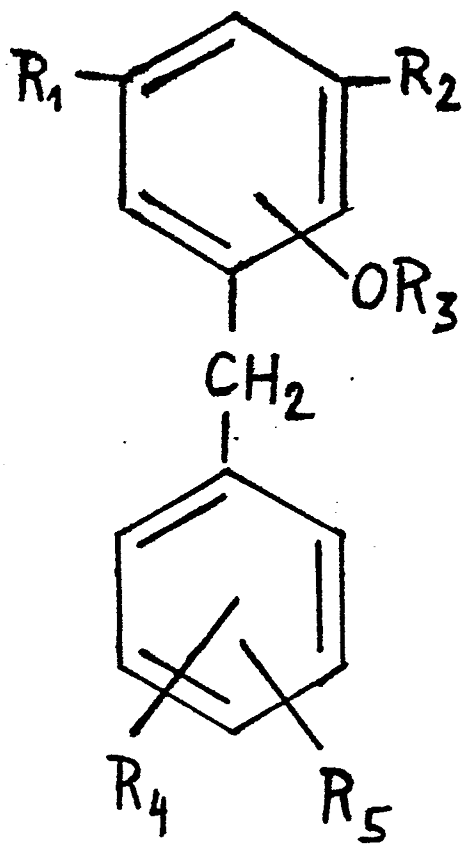

I